# EUROPEAN PATENT SPECIFICATION

(11) **EP 3 144 028 B1**
(45) Date of publication and mention of the grant of the patent: **31.01.2018**
(21) Application number: 16178087.9
(22) Date of filing: 06.07.2016
(51) Int. Cl.: A61M 25/10, A61B 17/22

(54) **BALLOON CATHETER**
BALLONKATHETER
CATHETER À BALLONNET

(30) Priority: 15.09.2015 JP 2015181288
(43) Date of publication of application: 22.03.2017
(73) Proprietor: ASAHI INTECC CO., LTD., Nagoya-shi, Aichi 463-0024 (JP)
(72) Inventor: KATSURADA, Takeharu, Nagoya-shi, Aichi 463-0024 (JP); KUBO, Yuta, Nagoya-shi, Aichi 463-0024 (JP); OGIDO, Moritaka, Nagoya-shi, Aichi 463-0024 (JP)
(74) Representative: Winter, Brandl, Fürniss, Hübner, Röss, Kaiser, Polte - Partnerschaft mbB

(56) References cited:
- WO-A1-2013/166209
- US-A- 5 429 605
- US-A1- 2002 082 549
- US-A1- 2008 004 568

## Description

### TECHNICAL FIELD

The present invention relates to a balloon catheter to be inserted into a stenosis site and the like in a blood vessel for expanding the stenosis site and the like.

### BACKGROUND

Balloon catheters to be inserted into a stenosis site and the like in a blood vessel for expanding the stenosis site and the like have already been proposed. A balloon catheter mainly comprises a balloon as an inflatable body; an outer shaft joined to the proximal end of the balloon; and an inner shaft inserted into the inside of the balloon and the outer shaft.

Various configurations have also been proposed in order to solve the problem that a balloon of a balloon catheter is detached from an inner shaft or an outer shaft at the joining portion therebetween when the balloon is inflated.

For example, US 6918920 discloses a balloon catheter in which a distal tip is joined to the distal end of an inner tube; a proximal shaft portion of a balloon is joined to the inner tube; and the inner tube, the distal tip and the proximal shaft portion of the balloon are joined by a sleeve.

Further, JP 2009-56297 A discloses a balloon catheter in which a proximal end of a balloon is sandwiched between a tube body and a heat-shrinkable tube.

However, in the foregoing balloon catheter according to US 6918920 , the proximal shaft portion of the balloon is merely joined to the outer periphery of the inner tube. Therefore, a problem may arise that the balloon tends to be detached from the inner tube when the balloon is inflated. Further, although the distal tip is joined to the distal end of the inner tube and the inner periphery of the sleeve, the joining strength is not enough. Therefore, disadvantageously, the distal tip also tends to break off from the inner tube. Specifically, when the internal pressure is increased in order to inflate the balloon, the proximal shaft portion of the balloon becomes susceptible to detachment from the inner tube. When the internal pressure is further increased, the balloon and the inner tube are forced to extend in the direction of the distal end. This causes the sleeve to be pushed from the proximal end, which, in turn, makes the sleeve susceptible to detachment, and the distal tip may break off from the inner tube.

Here, even if the foregoing heat-shrinkable tube disclosed in JP 2009-56297 A were used instead of the foregoing sleeve in US 6918920 , the problem that the distal tip may break off from the inner shaft could not be solved.

US 5429605, WO 2013/166209 A1 and US 2008/004568 A1 respectively disclose a balloon catheter in which a proximal end of a balloon is sandwiched between a tube body and an cover covering the distal end of the catheter.

### TECHNICAL PROBLEM

Starting from US 5429605, an objective of the present invention is to provide a balloon catheter in which a balloon is not easily detached from an inner shaft, and breakage and detachment of a tip from an inner shaft can be prevented without using a conventional sleeve and heat-shrinkable tube.

### SOLUTION TO PROBLEM

The present invention provides a balloon catheter comprising: a balloon, an inner shaft joined to the distal end of the balloon and a tip joined to the distal end of the inner shaft, wherein the tip extends in a direction of the proximal end so as to cover the outer periphery of the balloon, the distal end of the balloon, the inner shaft and the tip are joined to each other such that the distal end of the balloon is sandwiched between the inner shaft and a proximal end portion of the tip, an outer periphery of the inner shaft has unevenness at a joining portion in which the distal end of the balloon, the inner shaft and the tip are joined to each other, and the unevenness on the outer periphery of the inner shaft is formed with a coil body buried in the inner shaft .

According to the aforementioned configuration, the distal end of the balloon, the inner shaft and the tip are joined such that the distal end of the balloon is positioned at a portion sandwiched between the inner shaft and the proximal end portion of the tip. Therefore, the distal end of the balloon is not easily detached from the inner shaft. Further, since a conventional sleeve, heat-shrinkable tube and the like are not used, the number of components can be reduced as a whole. Furthermore, the joining strength between the tip and the balloon can be optimized by adjusting the length and area of the balloon covered with the tip. Therefore, a risk of breakage and detachment of the tip from the inner shaft can certainly be reduced.

Further, a configuration is proposed where the tip further comprises a tip distal end portion, a step portion and a tip proximal end portion, wherein the tip distal end portion extends from the step portion toward a distal end side of the balloon catheter, wherein the step portion makes contact with the distal end of the balloon and a distal end of the inner shaft, and wherein the tip proximal end portion extends from the step portion toward a proximal side of the balloon catheter.

Since the unevenness is formed on the outer periphery of the inner shaft at a place facing to the distal end of the balloon, the joining area between the distal end of the balloon and the outer periphery of the inner shaft can be increased. As a result, a configuration can be obtained in which the distal end of the balloon is not easily detached from the inner shaft. For example, the distal end of the balloon is caught in the unevenness formed on the outer periphery of the inner shaft by virtue of the anchor effect from the unevenness. Therefore, the distal end of the balloon is not easily detached from the inner-shaft even when a high pressure is applied to the balloon.

Since the unevenness is formed with the outer shape of the coil body as described above, an additional step of forming unevenness on the outer periphery of the inner shaft is not required. Therefore, the process for forming unevenness can be simplified.

Further, a configuration is proposed in which the inner periphery of the distal end of the balloon at the joining portion has unevenness corresponding to the unevenness of the outer periphery of the inner shaft.

According to the aforementioned configuration, the joining area between the distal end of the balloon and the outer periphery of the inner shaft can be further increased. As a result, a configuration can be obtained in which the distal end of the balloon is even more resistant to detachment from the inner shaft.

### ADVANTAGEOUS EFFECTS OF THE INVENTION

Advantageously, in the balloon catheter according to the present invention, a balloon is not easily detached from an inner shaft, and breakage and detachment of a tip from the inner shaft can be prevented.

### BRIEF DESCRIPTION OF THE DRAWINGS

Fig. 1 shows a partial cross sectional top view of the balloon catheter according to Example 1.
Fig. 2 shows an enlarged partial cross sectional view of the balloon catheter according to Example 1.
Fig. 3 shows an enlarged longitudinal sectional view in which disassembled components of the balloon catheter according to Example 1 are shown: (a) a tip, (b) an inner shaft and (c) a balloon.
Fig. 4 shows an enlarged partial cross sectional view of the balloon catheter according to Example 2 (Reference).
Fig. 5 shows an enlarged partial cross sectional view of the balloon catheter according to Example 3 (Reference).

### DESCRIPTION OF EMBODIMENTS

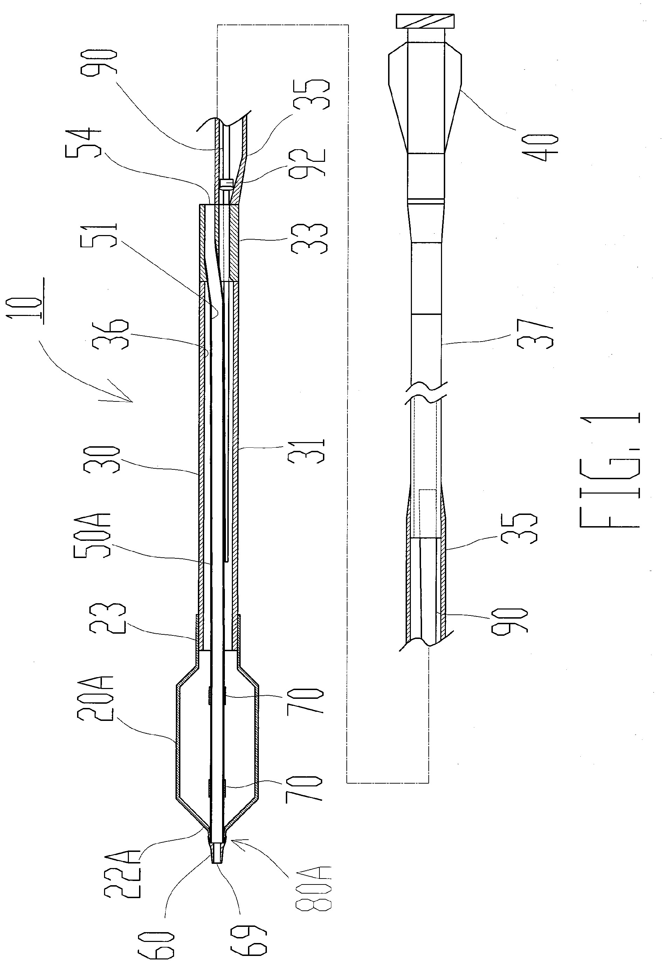

Below, Examples in which the balloon catheters according to the present invention are embodied will be described in detail. However, the present invention shall not be limited to Examples shown below, and modifications in design can be made appropriately. In Figs. 1 to 5, the left side corresponds to the distal end side which is to be inserted into the body, and the right side corresponds to the proximal side which is to be operated by an operator such as a physician.

### [Example 1]

For example, a balloon catheter 10 used for treating a stenosis site in a blood vessel of the heart comprises a balloon 20A, an outer shaft 30, a connector 40, an inner shaft 50A, a tip 60 and a core wire 90 as shown in Fig. 1.

The balloon 20A has a function to expand a stenosis site, and comprises a resin material. In addition, a distal end 22A of the balloon 20A is joined to the distal end of the inner shaft 50A and the tip 60, and a proximal end 23 of the balloon 20A is joined to the distal end of the outer shaft 30. Note that a joining structure of the distal end 22A of the balloon 20A will be described in more detail below.

The outer shaft 30 has a function to supply a fluid, and comprises a tubular member constituting an inflation lumen 36 for supplying the fluid. Further, the outer shaft 30 has a distal end outer shaft portion 31, a guide wire port portion 33, a middle outer shaft portion 35 and a proximal end outer shaft portion 37 in the order from the distal end side. Note that the guide wire port portion 33 corresponds to a portion where the distal end outer shaft portion 31, the middle outer shaft portion 35 and the inner shaft 50A are joined.

The inner shaft 50A is inserted into the distal end outer shaft portion 31, and the inflation lumen 36 is formed between the distal end outer shaft portion 31 and the inner shaft 50A. Further, the proximal end outer shaft portion 37 comprises a metal tubular member referred to as a so-called hypotube. In addition, the distal end of the proximal end outer shaft portion 37 is inserted into and joined to the proximal end of the middle outer shaft portion 35. Further, the connector 40 is attached to the proximal end of the proximal end outer shaft portion 37. Therefore, when a fluid such as a contrast agent and physiological saline for inflating the balloon 20A is supplied through an indeflator (not shown) to be attached to the connector 40, the fluid flows into the balloon 20A through the inflation lumen 36 to inflate the balloon 20A.

Note that the distal end outer shaft portion 31 and the middle outer shaft portion 35 are each preferably configured to be a tube comprising a resin such as polyamide, polyamide elastomer, polyolefine, polyester, or polyester elastomer. Further, the proximal end outer shaft portion 37 is preferably configured with stainless steel (SUS 304) or a superelastic alloy such as a Ni-Ti alloy.

The inner shaft 50A forms a guide wire lumen 51 for inserting a guide wire (not shown) thereinto. Further, the proximal end of the inner shaft 50A is joined to the guide wire port portion 33 of the outer shaft 30 to form a proximal end side guide wire port 54. Furthermore, a distal end side guide wire port 69 is formed at the tubular tip 60 arranged at the distal end of the inner shaft 50A.

Moreover, a marker member 70 having a cylindrical shape is arranged at the outer periphery of the inner shaft 50A in the inside of the balloon 20A.

The inner shaft 50A is preferably configured with a resin such as polyethylene, polyurethane, polyamide, polyamide elastomer, polyolefine, polyester or polyester elastomer. The tip 60 is preferably formed with a soft resin such as polyurethane, polyurethane elastomer. Moreover, the marker member 70 is preferably configured with a radiopaque metal material such as platinum and tungsten.

Further, the core wire 90 is attached to the inner periphery of the distal end of the proximal end outer shaft portion 37. The core wire 90 has a circular cross section, and configured with a tapered metal wire material with a diameter decreased toward the distal end. In addition, the core wire 90 is formed to extend so as to pass the middle outer shaft portion 35 and the guide wire port portion 33 through the distal end outer shaft portion 31. Further, the core wire 90 has a pressing member 92 near the proximal end of the guide wire port portion 33. By this, when a pushing force and a rotating force are applied to the core wire 90, the pressing member 92 makes contact with the guide wire port portion 33, allowing the pushing force and the rotating force to be transmitted to the distal end outer shaft portion 31 and the inner shaft 50A.

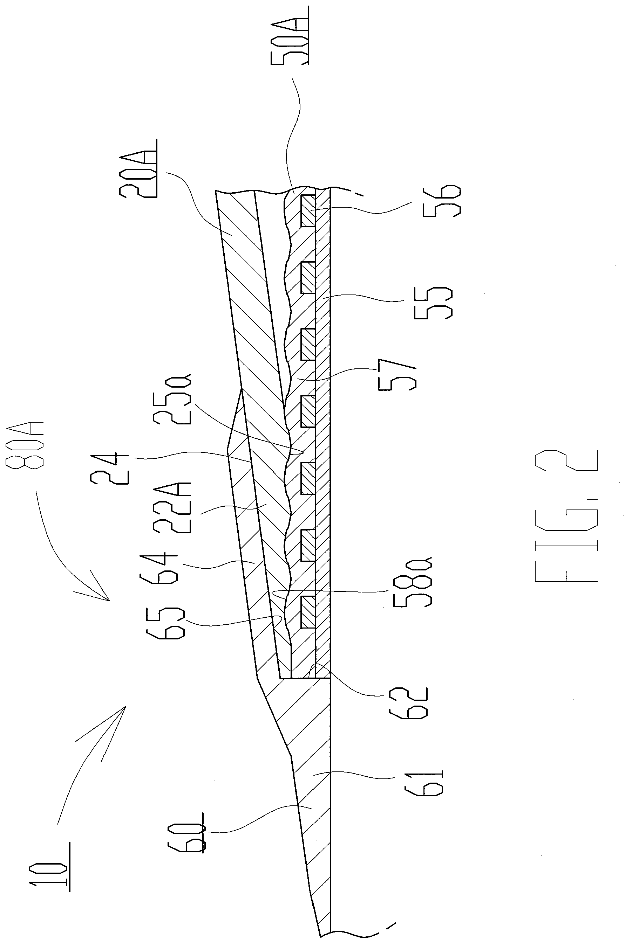

Below, the joining structure at the distal end 22A of the balloon 20A will be described with reference to Figs. 2 to 3C.

As shown in Fig. 2, the distal end 22A of the balloon 20A is sandwiched between the inner shaft 50A and the tip 60, and joined each other. In other words, the distal end 22A of the balloon 20A is inserted into a space between the inner shaft 50A and the tip 60, and fixed to the inner shaft 50A and the tip 60. According to the aforementioned joining structure, detachment of the distal end 22A of the balloon 20A from the inner shaft 50A can be prevented.

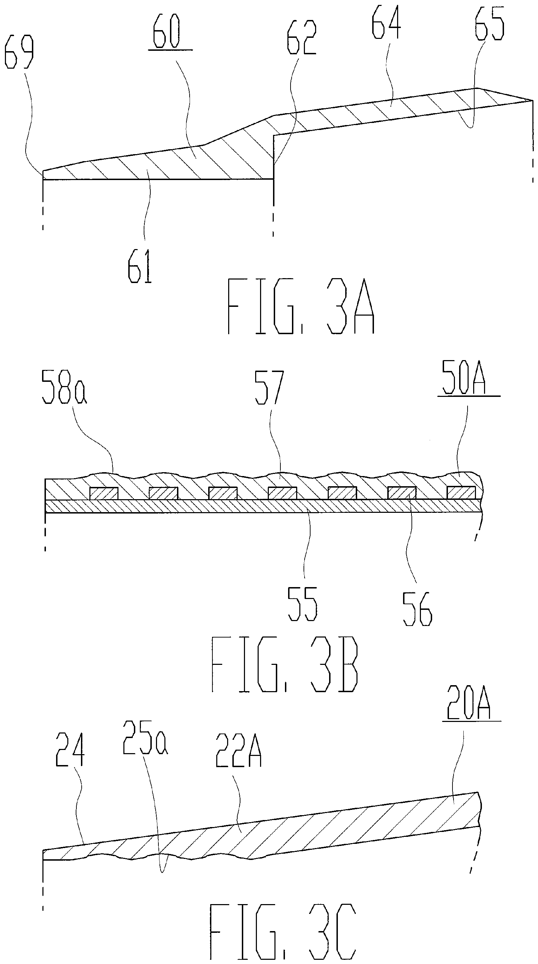

More specifically, as shown in Figs. 2 and 3A, a step portion 62 with which the distal end 22A of the balloon 20A and the distal end of the inner shaft 50A make contact is formed at the inner surface of the tip 60. Further, the tip 60 comprises a tip distal end portion 61 having a tapered shape and extending from the step portion 62 toward the distal end side guide wire port 69, and a tip proximal end portion 64 having a tapered shape and extending from the step portion 62 toward the proximal end. Note that the inner periphery of the tip proximal end portion 64 in the side of the inner shaft 50A corresponds to a tip proximal end joining inner surface 65. An inner periphery of the tip distal end portion 61 has a constant diameter from a distal end to a proximal end thereof. Minimum diameter of the inner periphery of the tip proximal end portion 64, where the step portion 62 is formed, is larger than the diameter of the inner periphery of the tip distal end portion 61. Diameter of the tip proximal end joining inner surface 65 gradually increases from the step portion 62 toward the proximal side end of the balloon catheter 10.

Furthermore, as shown in Figs. 2 and 3B, the inner shaft 50A has a tubular inner layer 55, a metal coil body 56 sparsely wound around the outer periphery of the inner layer 55 and an outer layer 57 formed over the coil body 56. Moreover, the outer shape of the coil body 56 buried into the inner shaft 50A forms unevenness on an outer periphery 58a at the distal end of the inner shaft 50A. In the example, the outer periphery 58a has a concave-convex surface.

Further, as shown in Figs. 2 and 3C, the inner periphery in the side of the inner shaft 50A at the distal end 22A of the balloon 20A corresponds to a balloon distal end joining inner surface 25a, and the unevenness is formed on the balloon distal end joining inner surface 25a. In addition, the balloon distal end joining inner surface 25a is pre-formed so as to correspond to the unevenness on the outer periphery 58a of the distal end of the inner shaft 50A.

Further, as shown in Fig. 2, in a state where the distal end of the inner shaft 50A and the distal end 22A of the balloon 20A make contact with the step portion 62 of the tip 60, the balloon distal end joining inner surface 25a of the distal end 22A of the balloon 20A makes surface contact with the outer periphery 58a of the inner shaft 50A on which unevenness is formed, and the outer periphery 24 of the distal end 22A of the balloon 20A is covered with the tip proximal end portion 64 of the tip 60, and the distal end 22A of the balloon 20A, the inner shaft 50A and the tip 60 are joined.

Here, a joining portion 80A is provided which corresponds to a portion where the distal end 22A of the balloon 20A, the inner shaft 50A and the tip 60 are joined each other. Therefore, even when a fluid is introduced into the inside of the balloon 20A to inflate the balloon 20A, and a stress is created for separating the distal end 22A of the balloon 20A from the distal end of the inner shaft 50A, detachment of the balloon 20A from the inner shaft 50A can be prevented because the distal end 22A of the balloon 20A is covered with the tip proximal end portion 64.

Further, the tip 60 is integrally formed from the tip distal end portion 61 of the distal end side guide wire port 69 through the tip proximal end portion 64. Therefore, breakage and detachment of the tip 60 from the inner shaft 50A can be prevented.

Moreover, the joining portion 80A described above comprises the balloon 20A, the inner shaft 50A and the tip 60, and other materials are not required. Therefore, the number of components does not increase. Further, since the tip 60 comprises the tip distal end portion 61 having a tapered shape and the tip proximal end portion 64 having a tapered shape, a stenosis site in a blood vessel can be expanded in a radial direction with the tip distal end portion 61 and the tip proximal end portion 64 when a balloon catheter 10 is inserted into the stenosis site. Further, the tip proximal end portion 64 covers the distal end 22A of the balloon 20A. Therefore, a risk of the balloon 20A damaged by the stenosis site can be reduced. As a result, when a fluid is introduced into the balloon 20A, a risk of rupture of the balloon 20A can be reduced.

Further, since the balloon distal end joining inner surface 25a of the balloon 20A and the outer periphery 58a of the inner shaft 50A each having corresponding unevenness make surface contact with each other, the anchor effect can be obtained as the joining area increases. Therefore, detachment of the distal end 22A of the balloon 20A from the inner shaft 50A can be much more effectively prevented.

Note that the unevenness on the outer periphery 58a of the inner shaft 50A is formed as follows: a molten resin is applied to the coil body 56 arranged on the inner layer 55, and then cured to form an outer layer 57 to which unevenness is exposed. Therefore, advantageously, an additional step for forming the unevenness is not required, and the number of manufacturing steps is not increased.

### [Example 2] (Reference)

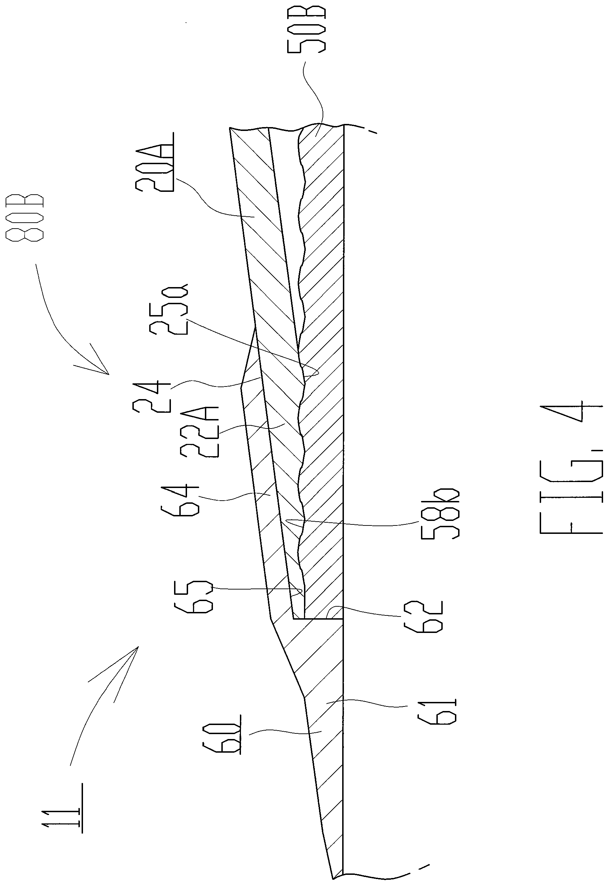

A joining portion 80B of a balloon catheter 11 according to Example 2 will be described with reference to Fig. 4. Note that the same reference numbers are assigned to structures similar to those in Example 1, and the descriptions thereof are omitted.

In Example 2, a monolayer inner shaft 50B is used instead of the inner shaft 50A used in Example 1, and the joining portion 80B comprises the tip 60, the distal end 22A of the balloon 20A and the inner shaft 50B. Further, unevenness is formed on the outer periphery 58b of the inner shaft 50B. Therefore, again according to the aforementioned configuration, detachment of the distal end 22A of the balloon 20A from the inner shaft 50B; breakage and detachment of the tip 60 from the inner shaft 50B; and damage to the balloon 20A upon inserted to a stenosis site can be reduced without increasing the number of components as in the balloon catheter 10 according to Example 1.

### [Example 3] (Reference)

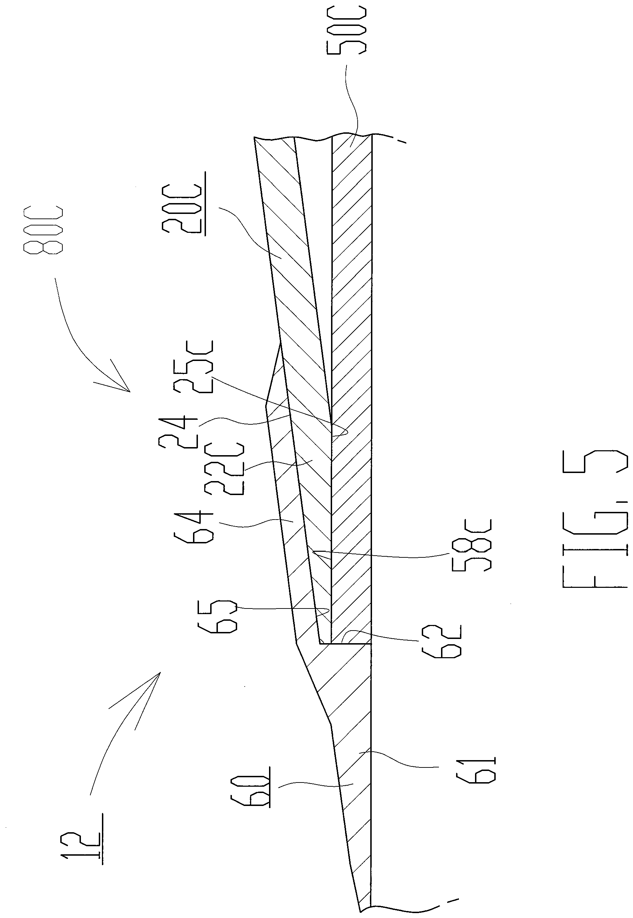

A joining portion 80C of a balloon catheter 12 according to Example 3 will be described with reference to Fig. 5. Note that the same reference numbers are assigned to structures similar to those in Examples 1 and 2, and the descriptions thereof are omitted.

In Example 3, a monolayer inner shaft 50C is used instead of the inner shaft 50A used in Example 1, and the joining portion 80C comprises the tip 60, a distal end 22C of a balloon 20C and the inner shaft 50C. Further, an outer periphery 58c of the inner shaft 50C is formed so as to have a smooth surface without unevenness. A balloon distal end joining inner surface 25c of the balloon 20C is also formed so as to have a smooth surface without unevenness. Therefore, again according to the aforementioned configuration, detachment of the distal end 22C of the balloon 20C from the inner shaft 50C; breakage and detachment of the tip 60 from the inner shaft 50C; and damage to the balloon 20C upon inserted to a stenosis site can be reduced without increasing the number of components as in the balloon catheter 10 according to Example 1.

Note that modifications can appropriately be made to the present invention in addition to Examples 1 to 3 as described above. For example, the unevenness on the outer periphery 58a, 58b of the inner shaft 50A, 50B shall not be limited to, for example, a coil-like unevenness along the shape of the coil body 56 wound sparsely. It may be those embossed in which a depressed portion and a protruded portion are provided at a given interval, or may be unevenness intermittently formed along the axial direction of the inner shaft 50A, 50B. Further, the unevenness on the outer periphery 58a of the inner shaft 50A may be formed with the coil body 56 completely buried in the inner shaft 50A, but the configuration is not limited to this. For example, (a) the outer periphery of the coil body 56 may be partially exposed to the outer layer 57 to allow the coil body 56 and the outer layer 57 to form unevenness on the outer periphery 58a of the inner shaft 50A, or (b) the unevenness on the outer periphery 58a of the inner shaft 50A may be formed with the coil body 56 and the inner layer 55 without using the outer layer 57.

Further, in the balloon catheter 10, 11, 12, a wide range of dimensions and shapes may suitably be selected for each component. Therefore, for example, the length of the tip proximal end portion 64 of the tip 60 along the axial direction of the tip 60, the thickness of the tip 60 in the radial direction and the like can be freely adjusted, thereby optimizing the joining strength between the tip 60, the distal end 22A, 22C of the balloon 20A, 20C and the inner shaft 50A, 50B, 50C.

Further, in Example 1, the coil body 56 having a rectangular cross sectional shape is wound around the outer periphery of the inner layer 55, but the configuration is not limited to this. The cross sectional shape of a wire of the coil body 56 may be circular, elliptical or polygonal. Further, the wire of the coil body 56 is not limited to a metal wire, and a wire comprising a resin such as PEEK (polyether ether ketone) may also be used. Furthermore, a braid in which multiple wires are woven in a mesh-like manner may also be used instead of the coil body 56.

### DESCRIPTION OF SYMBOLS

- 10, 11, 12: Balloon catheter
- 20A, 20C: Balloon
- 22A, 22C: Distal end
- 25a, 25c: Balloon distal end joining inner surface (inner periphery)
- 50A, 50B, 50C: Inner shaft
- 58a, 58b,: 58c Outer periphery
- 56: Coil body
- 60: Tip
- 80A, 80B, 80C: Joining portion

## Claims

1. A balloon catheter (10) comprising:
a balloon (20A);
an inner shaft (50A) joined to a distal end (22A) of the balloon (20A); and
a tip (60) joined to a distal end of the inner shaft (50A),
wherein the tip (60) extends in a direction of a proximal end so as to cover an outer periphery of the balloon (20),
wherein the distal end (22A) of the balloon (20A), the inner shaft (50A) and the tip (60) are joined to each other such that the distal end (22A) of the balloon (20) is sandwiched between the inner shaft (50) and a proximal end portion (64) of the tip (60), **characterized in that**
an outer periphery (58a) of the inner shaft (50A) has unevenness at a joining portion (80A) in which the distal end (22A) of the balloon (20A), the inner shaft (50A) and the tip (60) are joined to each other, and
the unevenness on the outer periphery (58a) of the inner shaft (50A) is formed with a coil body (56) buried in the inner shaft (50A).

2. The balloon catheter (10) according to claim 1,
the tip (60) further comprises a tip distal end portion (61), a step portion (62) and a tip proximal end portion (64),
wherein the tip distal end portion (61) extends from the step portion (62) toward a distal end side of the balloon catheter (10),
wherein the step portion (62) makes contact with the distal end (22A) of the balloon (20A) and a distal end of the inner shaft (50A), and
wherein the tip proximal end portion (64) extends from the step portion (62) toward a proximal side of the balloon catheter (10).

3. The balloon catheter (10) according to claim 1 or 2, wherein an inner periphery (25a) of the distal end (22A) of the balloon (20A) at the joining portion (80A) has unevenness corresponding to the unevenness of the outer periphery (58a) of the inner shaft (50A).

## Patentansprüche

1. Ballonkatheter (10) mit:
einem Ballon (20A);
einem an ein distales Ende (22A) des Ballons (20A) angefügten inneren Schaft (50A); und
einer an ein distales Ende des inneren Schafts (50A) angefügten Spitze (60),
wobei sich die Spitze (60) in Richtung eines proximalen Endes erstreckt und den Außenumfang des Ballons (20) umhüllt,
wobei das distale Ende (22A) des Ballons (20A), der innere Schaft (50A) und die Spitze (60) in der Weise aneinandergefügt sind, dass das distale Ende (22A) des Ballons (20) zwischen dem inneren Schaft (50) und einem proximalen Endabschnitt (64) der Spitze (60) angeordnet ist, **dadurch gekennzeichnet, dass**
der Außenumfang (58a) des inneren Schafts (50A) an einem Fügeabschnitt (80A), an dem das distale Ende (22A) des Ballons (20A), der innere Schaft (50A) und die Spitze (60) aneinandergefügt sind, eine Unebenheit aufweist, und
die Unebenheit am Außenumfang (58a) des inneren Schafts (50A) durch einen im inneren Schaft (50A) eingebetteten Wicklungskörper (56) geschaffen ist.

2. Ballonkatheter (10) nach Anspruch 1,
wobei die Spitze (60) des Weiteren einen distalen Spitzenendabschnitt (61), einen Stufenabschnitt (62) und einen proximalen Spitzenendabschnitt (64) aufweist,
wobei sich der distale Spitzenendabschnitt (61) von dem Stufenabschnitt (62) aus in Richtung einer distalen Seite des Ballonkatheters (10) erstreckt,
wobei der Stufenabschnitt (62) mit dem distalen Ende (22A) des Ballons (20A) und einem distalen Ende des inneren Schafts (50A) in Kontakt ist, und
wobei sich der proximale Spitzenendabschnitt (64) von dem Stufenabschnitt (62) aus in Richtung einer proximalen Seite des Ballonkatheters (10) erstreckt.

3. Ballonkatheter (10) nach Anspruch 1 oder 2, wobei der Innenumfang (25a) des distalen Endes (22A) des Ballons (20A) an dem Fügeabschnitt (80A) eine der Unebenheit des Außenumfangs (58a) des inneren Schafts (50A) entsprechende Unebenheit aufweist.

## Revendications

1. Cathéter à ballonnet (10) comprenant :
un ballonnet (20A) ;
un arbre interne (50A) joint à une extrémité distale (22A) du ballonnet (20A) ; et
un embout (60) joint à une extrémité distale de l'arbre interne (50A),
dans lequel l'embout (60) s'étend dans une direction d'une extrémité proximale de façon à recouvrir une périphérie externe du ballonnet (20),
dans lequel l'extrémité distale (22A) du ballonnet (20A), l'arbre interne (50A) et l'embout (60) sont joints les uns aux autres de telle sorte que l'extrémité distale (22A) du ballonnet (20) est intercalée entre l'arbre interne (50) et une partie extrémité proximale (64) de l'embout (60), **caractérisé en ce que**
une périphérie externe (58a) de l'arbre interne (50A) a une irrégularité au niveau d'une partie de jonction (80A) dans laquelle l'extrémité distale (22A) du ballonnet (20A), l'arbre interne (50A) et l'embout (60) sont joints les uns aux autres, et
l'irrégularité de la périphérie externe (58a) de l'arbre interne (50A) est formée d'un corps de bobine (56) enfoui dans l'arbre interne (50A).

2. Cathéter à ballonnet (10) selon la revendication 1,
l'embout (60) comprenant en outre une partie extrémité distale d'embout (61), une partie palier (62) et une partie extrémité proximale d'embout (64),
dans lequel la partie extrémité distale d'embout (61) s'étend depuis la partie palier (62) vers un côté extrémité distale du cathéter à ballonnet (10),
dans lequel la partie palier (62) établit le contact avec l'extrémité distale (22A) du ballonnet (20A) et une extrémité distale de l'arbre interne (50A), et
dans lequel la partie extrémité proximale d'embout (64) s'étend depuis la partie palier (62) vers un côté proximal du cathéter à ballonnet (10).

3. Cathéter à ballonnet (10) selon la revendication 1 ou 2, dans lequel une périphérie interne (25a) de l'extrémité distale (22A) du ballonnet (20A) au niveau de la partie de jonction (80A) a une irrégularité correspondant à l'irrégularité de la périphérie externe (58a) de l'arbre interne (50A).
